# EUROPEAN PATENT SPECIFICATION

(11) **EP 2 277 503 B1**
(45) Date of publication and mention of the grant of the patent: **12.02.2014**
(21) Application number: 10010775.4
(22) Date of filing: 06.03.2003
(51) Int. Cl.: A61K 9/00, A61K 31/426, A61K 31/341, A61K 31/4439

(54) **Effervescent compositions comprising bisphosphonates**
Brausetabletten, enthaltend Bisphosphonate
Compositions effervescentes comprenant des bisphosphonates

(30) Priority: 06.03.2002 US 92083
(43) Date of publication of application: 26.01.2011
(62) Divisional of application: 03716287.2
(73) Proprietor: EffRx Pharmaceuticals SA, 1005 Lausanne (CH)
(72) Inventor: McCallister, David, Palm Beach Gardens, FL 33410 (US); Rosen, Christer, Tequesta, FL 33469 (US)
(74) Representative: Mai, Dörr, Besier

(56) References cited:
- WO-A-97/44017
- WO-A2-01/39724
- GB-A- 2 153 225
- US-A- 5 393 531
- US-A- 5 415 870
- US-A1- 2001 002 395
- GERTZ B J ET AL: "STUDIES OF THE ORAL BIOAVAILABILITY OF ALENDRONATE", CLINICAL PHARMACOLOGY & THERAPEUTICS, MOSBY-YEAR BOOK, ST LOUIS, MO, US, vol. 58, no. 3, September 1995 (1995-09), pages 288-298, XP001006243, ISSN: 0009-9236

## Description

### TECHNICAL FIELD OF THE INVENTION

The present invention relates generally to effervescent compositions. More particularly, the invention pertains to effervescent compositions comprising a bisphosphonate, and is particularly useful in the treatment of osteoporosis.

### BACKGROUND OF THE INVENTION

Osteoporosis is a disease that leads to defective skeletal function caused by low bone mass and deterioration of bone quality. In this respect, bone characterized by weakened mechanical strength is much more susceptible to fracture, even under stresses that would otherwise be tolerated by normal bone. In the United States alone, some 8 million women and 2 million men suffer from osteoporosis. Many more are at increased risk for osteoporosis because they have low bone mass. The risk of suffering from osteoporosis increases with age, particularly in those over the age of 50.

Osteoporosis results from a disorder in bone remodeling, which is the process by which bone tissue is continually renewed and repaired. In bone remodeling, osteoclasts resorb old or damaged bone, while osteoblasts synthesize a new bone matrix. In patients who suffer from osteoporosis, the rate of bone resorption undesirably exceeds the rate of bone formation, either because too much bone is resorbed or too little bone is formed.

Drugs used in the treatment of osteoporosis act by inhibiting bone resorption. Among such anti-resorptive agents are bisphosphonate compounds. Bisphosphonates are synthetic analogues of pyrophosphates, which are naturally occurring regulators of bone turnover. 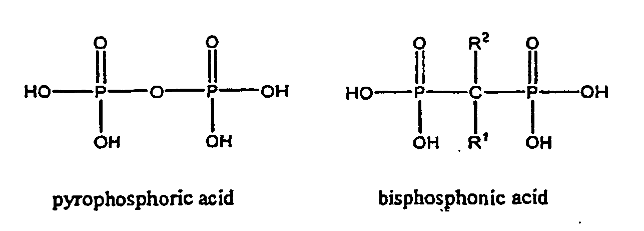 Bisphosphonates, like pyrophosphates, can bind to the surface of the hydroxyapatite bone matrix. It will be appreciated by the ordinarily skilled artisan that the pharmacologic properties of bisphosphonates can be varied through side chain substitutions (R¹, R²) on their general chemical structure. For example, alendronate has an amino side chain (R² = CH₂CH₂CH₂NH₂) while risedronate has a cyclic nitrogen-containing side chain. The R¹ and R² side chains for a variety of bisphosphonate compounds are given in Table 1.

**Table 1**

| Bisphosphonate | R¹ side chain | R² side chain |
|---|---|---|
| Etidronate | OH | CH₃ |
| Clodronate | C1 | CI |
| Pamidronate | OH | CH₂CH₂NH₂ |
| Alendronate | OH | (CH₂)₃NH₂ |
| Risedronate | OH | CH₂-3-pyridyl |
| Tiludronate | H | CH₂-S-phenyl-C1 |
| Ibandronate | OH | CH₂CH₂N(CH₃)(pentyl) |
| Zoledronate | OH | CH₂-imidazole |
| YH529 | OH | CH₂-2-imidazopyridinyl |
| Incadronate | H | N-(cycloheptyl) |
| Olpadronate | OH | CH₂CH₂N(CH₃)₂ |
| Neridronate | OH | (CH₂)₅NH₂ |
| EB-1053 | OH | CH₂-1-pyrrolidinyl |

The oral administration of bisphosphonate compounds for the treatment of osteoporosis suffers from several major drawbacks. First, bisphosphonate compounds are very poorly absorbed from the gastrointestinal tract into the blood of a patient (e.g., have low bioavailability). In fact, typically only about 0.5% to about 5% of the total bisphosphonate active ingredient is absorbed from an oral dosage formulation (e.g., a solid tablet). Once in the blood stream, typically only about 20% to about 50% of the bisphosphonate becomes bound to the bone surface. The bioavailability of bisphosphonate is further reduced under highly acidic gastric conditions, which occur when the patient has eaten food or consumed an acidic beverage (e.g., coffee, tea, or orange juice).

The bioavailability of the bisphosphonate is further affected by the delivery system. Solid delivery systems such as capsules and tablets must be ingested with sufficient liquid to disintegrate the dosage form. Once the dosage form is inside the stomach or small intestine of a patient, it has to disintegrate into small particles, and the active ingredient has to be solubilized such that it can be absorbed into the plasma of the patient. The disintegration and solubilization processes for solid dosage forms delay the bioavailability of the active ingredient.

Effervescent drug formulations can offer enhanced dissolution and absorption of active ingredients resulting in increased bioavailability. Frequently, active ingredients are absorbed better from effervescent formulations as compared to dry, solid tablet formulations. Effervescent tablets also can be larger in size allowing for higher drug loading as well as combination drug loading. Because the tablets are dissolved in water, they are easier to swallow than dry solid tablet formulations. Furthermore, effervescent compositions can be formulated without polyvalent metal ions (e.g., Ca²⁺ and Mg²⁺), which can bind to bisphosphonate compounds rendering them insoluble and unabsorbable. An effervescent bisphosphonate composition is disclosed in U.S. Patent 5,853,759 which describes an effervescent tablet comprising a bisphosphonate, in particular alendronate, an acid component, and an alkaline effervescing component.

A second drawback, even with effervescent formulation, often encountered by patients taking regular dosages of bisphosphonates is the common occurrence of upper gastrointestinal disturbances. Such disturbances include heartburn, esophageal irritation, and even, in some cases, esophageal ulcers. The local tissue irritation and ulceration associated with the administration of bisphosphonates may be mitigated by the administration of drugs that suppress gastric acid production. Anti-ulcer agents such as H₂-antagonists (i.e., histamine H₂-receptor antagonists) and proton pump inhibitors are known to be effective in combating acid-peptic diseases. Proton pump inhibitors (e.g., H⁺, K⁺-ATPase inhibitors) are α -pyridylmethylsulfinyl benzimidazole compounds having different pyridine or benzimidazole substituents. The proton pump inhibitors react with acid (are activated) to form thiophilic sulfenamide or sulfenic acid components. Once activated, the compounds irreversibly bind to the sulfhydryl group of cysteine residues thus halting acid production. Commercially available H₂-antagonists include cimetidine (Tagamet®), ranitidine (Zantac®), famotidine (Pepcid®), and nizatidine (Axid®). The H₂-antagonists inhibit acid production by competing with histamine in binding to H₂-receptors.

The combination of these problems has led to a complicated regimen that attempts to optimize bioavailability of the bisphosphonate while minimizing the gastrointestinal problems. Thus, current regimens require the patient to (a) remain strictly upright for at least 30 minutes after taking the bisphosphonate composition so as to minimize esophageal irritation, and (b) wait as much as 2 hours before eating.

U.S. Patents 5,994,329, 6,015,801, 6,225,294, and 6,333,316 as well as EP 1 127 573 Al disclose methods for inhibiting bone resorption comprising sequential administration of histamine H₂ receptor blockers and/or proton pump inhibitors about 30 minutes to about 24 hours prior to administration of bisphosphonates. Administration of such histamine H₂ receptor blockers and/or proton pump inhibitors in this manner exacerbates the already complicated and inconvenient regimen because the patient would have to take the histamine H₂ receptor blockers and/or proton pump inhibitors (e.g., upon waking), then wait 30 minutes or longer to take the bisphosphonate and then wait up to 2 more hours to eat.

Despite the availability of the foregoing approaches, it will be appreciated that there remains a need in the art for compositions and drug administration regimens for osteoporosis, or other bone resorption disorders, which are less complicated and more convenient than those commonly known in the art. Moreover, there remains a need for a composition that can deliver a bisphosphonate drug for treating osteoporosis (or other bone resorption disorders) while reducing associated ulceration. The invention seeks to provide such compositions to satisfy at least one of these needs. These and other advantages of the invention will be apparent from the description of the invention provided herein.

### BRIEF SUMMARY OF THE INVENTION

The invention provides an effervescent composition comprising a bisphosphonate, which upon dissolution in water has a buffered pH. In accordance with the present invention, it has been found that including a bisphosphonate in such an effervescent composition imparts improved bioavailablity of the bisphosphonate to the body of a mammal. Desirably, the effervescent composition of the invention, when orally administered to a mammal, preferably raises the pH of the gastric juices of the stomach of the mammal to a pH of 3 or more.

Thus, in one aspect, the present invention provides an effervescent composition comprising a bisphosphonate, an acid component, and an alkaline effervescing component such that the composition, when dissolved in water, produces a solution having a buffered pH of 3 to 6.5.

In another aspect, the present invention provides an effervescent composition that comprises a bisphosphonate, an anti-ulcer agent, an acid component, an alkaline effervescing component, and optionally a sweetener, a flavorant, or a solubilizing agent.

In yet another aspect, the present invention provides an effervescent composition that comprises a microencapsulated bisphosphonate, an acid component, an alkaline effervescing component, and optionally an anti-ulcer agent.

The administration of a bisphosphonate in an effervescent composition to a mammal, in accordance with the present invention, results in improved bioavailability of the bisphosphonate compared to conventional non-effervescent bisphosphonate formulations. As such, if desired, the present invention advantageously can allow for providing a single effervescent composition for delivering the bisphosphonate in combination with an anti-ulcer agent. Furthermore, the bisphosphonate can be present in quantities that are larger than those provided by conventional non-effervescent solid formulations that must be able to be swallowed whole.

The present invention may be best understood with reference to the following detailed description of the preferred embodiments.

### DETAILED DESCRIPTION OF THE INVENTION

The present invention is predicated, at least in part, on the surprising and unexpected discovery that a buffered effervescent composition, comprising a bisphosphonate, an acid component, and an alkaline effervescing component, and having a pH of 3 to 6.5 when dissolved in water, exhibits improved absorption, and thus improved bioavailability, of the bisphosphonate in the body. In this respect, it is believed that the effervescent composition provides an acid mediating effect on the stomach pH such that the bisphosphonate is more readily absorbed from the intestinal tract into the blood stream.

It has further been found that effervescent compositions comprising both a bisphosphonate and an anti-ulcer agent further enhance the absorption of the bisphosphonate. Pursuant to the present invention, the term "anti-ulcer agent" is defined as including H₂ antagonists and/or proton pump inhibitors.

Thus, the effervescent composition according to the invention comprises a bisphosphonate in the inventive effervescent composition and, if desired, may further comprise an anti-ulcer agent. In some embodiments, the effervescent composition comprises a microencapsulated bisphosphonate alone or in combination with an anti-ulcer agent.

The bisphosphonate can be any suitable bisphosphonate. For example, the bisphosphonate can be (4-amino-1-hydroxybutylidene)bisphosphonate (alendronate), [(cycloheptylamino)methylene]bisphosphonate (cimadronate), (dichloromethylene )bisphosphonate (clodronate, [1-hydroxy-3-(1-pyrrolidinyl)- propylidene ]bisphosphonate (EB-1053), (1-hydroxyethylidene)bisphosphonate (etidronate), [1-hydroxy-3-(methylpentylamino )propylidene ]bisphosphonate (ibandronate), (6-amino-1-hydroxyhexylidene )bisphosphonate (neridronate ), [3-(dimethylamino)-1-hydroxypropylidene]bisphosphonate (olpadronate), (3-amino-1-hydroxypropylidene)bisphosphonate (pamidronate), [1-hydroxy-2-(3-pyridinyl)ethylidene]bisphosphonate (risedronate), [[(4-chlorophenyl)thio]methylene] bisphosphonate (tiludronate), [1-hydroxy-2-(imidazo-(1,2a)pyridin-3-yl)ethylidene ]bisphosphonate (YH 529), [1-hydroxy-2-(1H-imidazol-1-yl)ethylidene]bisphosphonate (zoledronate), or a combination thereof. Preferably, the bisphosphonate is selected from the group consisting of etidronate, risedronate, alendronate, and combinations thereof. More preferably, the bisphosphonate is etidronate.

Typically, 0.25% or more ( e.g., 1% or more or 2% or more) bisphosphonate, based on the total weight of the composition, is present in the effervescent composition. For example, the effervescent composition can comprise 0.25% to 33.3% bisphosphonate, based on the total weight of the composition. In some embodiments, 1.5% or more (e.g., 3% or more or 5% or more) and 30% or less (e.g., 20% or less) bisphosphonate, based on the total weight of the composition, is present in the effervescent composition. The actual amount of bisphosphonate present in the effervescent composition depends on the specific bisphosphonate chosen. When the bisphosphonate is etidronate, typically 70 mg or more and 2000 mg or less bisphosphonate is present. In some embodiments, 100 mg to 500 mg (e.g., 200 mg to 400 mg) etidronate is present in the effervescent composition.

The bisphosphonate can be finely milled to enhance the absorption of the bisphosphonate. In this respect, such finely milled particles of the bisphosphonate result in more surface area being exposed to the absorption medium, thereby maximizing absorption. Desirably, the bisphosphonate is milled to a particle size of 100 microns or less (e.g., 4 microns to 100 microns). Preferably, the bisphosphonate is milled to a particle size of 5 microns to 70 microns (e.g., 10 microns to 50 microns).

The acid component can be any suitable acid component. Typically, the acid component is an organic or mineral acid that is safe for consumption and which provides for effective and rapid effervescent disintegration upon contact with water and the alkaline effervescent component. For example, the acid component can be selected from the group consisting of citric acid, tartaric acid, malic acid, fumaric acid, adipic acid, succinic acid, acid anhydrides, acid salts, mixtures of acid salts; acid salts of disodium dihydrogen pyrophosphate, acid citrate salts and other related organic acids and their salts, and combinations thereof. Preferably, the acid component it citric acid (e.g., citric acid anhydrous) or tartaric acid.

The alkaline effervescing component can be any suitable alkaline effervescent component. Typically, the alkaline effervescing component is an organic or mineral based (e.g., alkali metal carbonate) that is safe for consumption and provides effective and rapid effervescent disintegration upon contact with water and the acid component For example, the alkaline effervescing component can be selected from the group consisting of carbonate salts, bicarbonate salts, and mixtures thereof. Preferably, the alkaline effervescing component is selected from the group consisting of sodium bicarbonate, sodium carbonate anhydrous, potassium carbonate, and potassium bicarbonate, sodium glycine carbonate, calcium carbonate, calcium bicarbonate, L-lysine carbonate, arginine carbonate, and combinations thereof. In some embodiments, the alkaline effervescing component is sodium bicarbonate, potassium bicarbonate, sodium carbonate, or a combination thereof. The use of non-sodium alkaline effervescing components is desirable for use with patients requiring electrolyte maintenance, e.g., hypertensive and other cardiac patients.

The acid component and the alkaline effervescent component can be present in the effervescent composition in any suitable amount. The amounts of acid component and alkaline effervescent component are such that the effervescent composition, when dissolved in water, produces a buffer solution. For the purposes of clarity, by the phrase "effervescent composition completely dissolves" it is meant that the acid component and the alkaline effervescent component are completely dissolved in the water, even though another components (e.g., the bisphosphonate or a microencapsulated bisphosphonate) are not completely dissolved or not at all dissolved. The buffer solution is produced when the acid component reacts with the alkaline effervescent component to produce a solution containing the acid component and/or the alkaline effervescent component in equilibrium with the fully deprotonated salt of the acid component. For example, when the acid component is citric acid and the alkaline component is sodium bicarbonate, the buffer would consist of an acid form of citric acid and sodium citrate (e.g., the fully deprotonated salt of citric acid). Typically, the buffered solution has a pH of 3 to 6.5 (e.g., 3.5 to 6.5 or 4 to 6.5). Preferably, the buffered solution has a pH of 3.5 to 6 (e.g., 4 to 6). More preferably, the buffered solution has a pH of 4 to 5.5 (e.g., 4.5 to 5.5). For example, in some embodiments the buffered solution contains an amount of fully deprotonated salt of the acid component that is at least 1.5 times (e.g., at least 1.75 times or at least 2 times) the amount of acid equivalents ( e.g., equivalents of acid groups within the acid component). In some embodiments, the amount of acid equivalents is non-zero.

The buffer produced upon dissolution of the effervescent composition in water is sufficient to raise the pH of a patient's stomach upon passage of the solution thereinto. The pH of the gastric juices in the stomach of the patient preferably is raised to a pH of 3 or greater (e.g., 3.5 or greater), more preferably 4 or greater (e.g., 4.5 or greater). The buffer solution typically is capable of mediating the pH of a patient's stomach for at least 10 minutes or more (e.g., 15 minutes or more). In some embodiments, the buffer solution is capable of mediating the pH of a patient's stomach for up to 30 minutes or more (e.g., up to 45 minutes or more).

For example, the acid component can be present in the effervescent composition in an amount of from 15% to 60% (e.g., 25% to 45%) based on the total weight of the composition. Typically, 800 mg to 1600 mg acidic compound is present in the effervescent composition. The alkaline effervescent component is present in the effervescent composition, for example, in an amount of from 20% to 70% (e.g., 35% to 55%) based on the total weight of the composition. For example, 800 to 1600 mg sodium bicarbonate, 0 mg to 800 mg potassium bicarbonate, and 20 mg to 100 mg sodium carbonate can be present in the effervescent composition.

In one embodiment, the effervescent composition optionally further comprises an anti-ulcer agent. The anti-ulcer agent can be an H₂-antagonist, a proton pump inhibitor, or a combination thereof. The H₂-antagonist can be any suitable H₂-antagonist. For example, the H₂-antagonist can be ranitidine, cimetidine, famotidine, nizatidine, or a combination thereof. Preferably, the H₂-antagonist is ranitidine or cimetidine. Typically, the H₂-antagonist is present in the effervescent composition in an amount of 3.3% to 57.5% (e.g., 8% to 40%) based on the total weight of the composition. The H₂-antagonist preferably is present in the effervescing composition in an amount of 5 mg to 500 mg, depending on the specific antagonist selected. By way of example, when the H₂-antagonist is ranitidine, the composition comprises 25 mg to 300 mg ( e.g., 50 mg to 200 mg) H₂-antagonist; when the H₂-antagonist is cimetidine, the composition comprises 25 mg to 400 mg ( e.g., 50 mg to 350 mg) H₂-antagonist; when the H₂-antagonist is famotidine, the composition comprises 2 mg to 50 mg ( e.g., 5 mg to 30 mg) H₂-antagonist; and when the H₂-antagonist is nizatidine, the composition comprises 25 mg to 350 mg (e.g., 50 to 300 mg) H₂-antagonist.

The anti-ulcer agent comprises any suitable proton pump inhibitor. By way of example, the proton pump inhibitor can be omeprazole, lansoprozole, rabeprazole, pantoprazole, or a combination thereof. Preferably, the proton pump inhibitor is omeprazole. Typically, the proton pump inhibitor is present in an amount of from 0.5% to 60% by weight of the composition (e.g., 5% to 40%). The proton pump inhibitor is present in the effervescing composition in an amount of 5 mg to 100 mg, depending on the specific proton pump inhibitor used. For example, when the proton pump inhibitor is omeprazole, the composition comprises 10 mg to 30 mg proton pump inhibitor; when the proton pump inhibitor is lansoprazole, the composition comprises 2 mg to 30 mg (e.g., 5 mg to 20 mg) proton pump inhibitor; when the proton pump inhibitor is rabeprazole, the composition comprises 5 mg to 60 mg (e.g., 10 to 45 mg) proton pump inhibitor; and when the proton pump inhibitor is pantoprazole, the composition comprises 5 mg to 50 mg (e.g., 10 mg to 35 mg) proton pump inhibitor.

When the effervescent composition comprises one of the anti-ulcer agents described above, the capacity of the buffer solution to mediate the pH of a patient's stomach typically is extended beyond that of the effervescent composition without the anti-ulcer agent. For example, the dissolved solution is able to mediate the pH of a patient's stomach for up to 60 minutes or more (e.g., up to 90 minutes or more or up to 120 minutes or more).

The effervescent composition optionally further comprises a solubilizing agent, which aids in the transition of the bisphosphonate from the gastrointestinal tract to the blood by solubilizing the bisphosphonate and facilitating its transfer into the mucosal interface of the gastrointestinal tract. The solubilizing agent can be any suitable solubilizing agent. For example, the solubilizing agent can be a polyvinylpyrrolidone, a polyethylene glycol, a dextran, or a combination thereof. The polyvinylpyrrolidone and polyethylene glycol can have any suitable molecular weight. For example, the polyvinylpyrrolidone can have a molecular weight of 20,000 g/mol to 40,000 g/mol, preferably 25,000 g/mol to 35,000 g/mol, more preferably 28,000 g/mol to 32,000 g/mol. The polyethylene glycol can have a molecular weight of 2000 g/mol to 10,000 g/mol (e.g., 4000 g/mol, 6000 g/mol, or 8000 g/mol). The dextran can be any suitable branched poly-D-glucoside having predominantly C₁₋₆ glycosidic bonds. In some embodiments, the solubilizing agent comprises both polyvinylpyrrolidone and polyethylene glycol. Typically, the solubilizing agent is present in the effervescent composition in an amount of from 0.1 % or more based on the weight of the composition. For example, 0.1% to 10% (e.g., 1% to 5%) based on the weight of the composition polyvinylpyrrolidone and/or 20 mg to 100 mg polyethylene glycol can be present in the effervescent composition. When the solubilizing agent is a dextran, 1% to 20%, preferably 5% to 15% (e.g., 10% ) dextran based on the weight of the composition can be present in the effervescent composition.

The effervescent compositions described above optionally further comprise a sweetener and/or a flavorant. In some embodiments, the effervescent composition comprises both a sweetener and a flavorant. The sweetener can be any suitable natural or artificial sweetener or a combination of natural and artificial sweeteners. Acceptable natural sweeteners include members selected from the group of glucose, dextrose, invert sugar, fructose, glycyrrhizic acid, and mixtures thereof. Typically, the natural sweetener is present in the effervescent composition in an amount of 10% to 50% (e.g., 20% to 40%) based on the weight of the composition. Acceptable artificial sweeteners include members selected from the group consisting of saccharin; aspartame; chloro-derivatives of sucrose such as sucralose, cyclamate, acesulfame-K; sugar alcohols such as sorbitol, mannitol, and xylitol; and mixtures thereof. Typically, the artificial sweetener is present in the effervescent composition in an amount of 0 to 5% ( e.g., 0.1 to 2.5%) based on the weight of the composition. Preferably, the sweetener is an artificial sweetener selected from the group consisting of aspartame, saccharin, acesulfame-K, xylitol, Splenda®, and combinations thereof. The effective concentration of a sweetener is determined by the strength of its sweetness, solubility, and masking ability for a specific active ingredient(s). For example, when the sweetener is aspartame, 10 mg to 50 mg aspartame is present in the effervescent composition. When the sweetener is saccharin, 10 mg to 30 mg saccharin is present in the effervescent composition. When the sweetener is acesulfame-K, 10 mg to 50 mg acesulfame-K is present in the effervescent composition. When the sweetener is xylitol, 100 mg to 400 mg xylitol is present in the effervescent composition. When the sweetener is Splenda®, 10 mg to 50 mg Splenda® is present in the effervescent composition.

The flavorant can be a natural or synthetic flavorant. Typically, the flavorant is present in the effervescent composition in an amount of 0% to 10% (e.g., 1% to 7.5%) based on the weight of the composition. Acceptable flavorants include members selected from the group consisting of volatile oils, synthetic flavor oils, oleoresins, plant extracts (e.g., green tea flavor), and combinations thereof. Desirable flavorants include a member selected from the group consisting of citrus oils such as lemon, orange, grape, lime and grapefruit; fruit essences such as apple, pear, peach, grape, strawberry, raspberry, cherry, plum, pineapple, and apricot; and other fruit flavors. Other useful flavorants include aldehydes and esters such as benzaldehyde (cherry, almond), citral (lemon, lime), neral (lemon, lime), decanal (orange, lemon), C₈-aldehyde (citrus fruits), C₉- aldehyde (citrus fruits), C₁₂-aldehyde (citrus fruits), tolylaldehyde (cherry, almond), 2,6-dimethyloctanal (green fruit), and 2-dodecenal (citrus, mandarin), and mixtures thereof Preferably, the flavorant is cherry, citrus, or orange. The flavorant typically is present in the effervescent composition in an amount of 10 mg to 100 mg.

In one embodiment, the effervescent composition comprises a bisphosphonate, an anti-ulcer agent, an acid component, an alkaline effervescing component, and, optionally, one or more ingredients selected from sweeteners, flavorants, and solubilizing agents. Typically, the effervescent composition comprises 3% to 19% bisphosphonate, 0.5% to 50% anti-ulcer agent, 15% to 60% acid component, 20% to 70% alkaline effervescing component, 0% to 5% sweetener, 0% to 10% flavorant, and 0% to 10% solubilizing agent. Preferably, the effervescent composition comprises 5% to 15% bisphosphonate, 2% to 30% anti-ulcer agent, 25% to 45% acid component, 30% to 60% alkaline effervescing component, 2% to 4% sweetener, 3% to 8% flavorant, and 1% to 5% solubilizing agent. The percent amounts recited above are based on the total weight of the effervescent composition.

In another embodiment, the effervescent composition comprises a bisphosphonate compound that is encapsulated (e.g., microencapsulated) in a time-release (e.g., sustained-, delayed- or directed-release) delivery system. The encapsulation of the bisphosphonate is preferred when delayed release of the active ingredient into the stomach is desired, for example, when the bisphosphonate is administered simultaneously with an H₂-antagonist or a proton pump inhibitor. Encapsulation of the bisphosphonate could minimize or eliminate irritation of the esophagus of the patient and other patient distress such as nausea. Encapsulation of the bisphosphonate also could delay release of the bisphosphonate until the stomach absorbs the anti-ulcer agent and inhibition of the stomach's acid production has begun. The encapsulation could delay release of the bisphosphonate by 2 to 30 minutes, preferably by 5 to 10 minutes. It is to be noted that effervescent compositions comprising encapsulated bisphosphonate, when dissolved in water, achieve the desired buffered pH in accordance with the invention. In this respect, the acid component and the alkaline effervescent component are completely dissolved but, of course, the encapsulated bisphosphonate remains encapsulated.

The bisphosphonate can be encapsulated in any suitable manner as will be readily appreciated by one of ordinary skill in the art. For example, the polymer coating can be any suitable differentially degrading coating including microencapsulation, enteric coating, multiple coating, and the like. The polymer coating may be one that resists disintegration upon contact with the saliva but instantly releases the bisphosphonate upon contact with gastric juice in the stomach. Alternatively, the polymer coating may be one that resists rapid disintegration in the presence of gastric juice. Suitable coating polymers include biodegradable polymers such as polylactic acid, polyglycolic acid, copolymers of lactic and glycolic acid, polyorthoesters, and polyanhydrides thereof. The bisphosphonate also can be encapsulated by a polymer coating such as a cellulosic (e.g., methyl or ethyl cellulose) coating, a wax coating, a gum coating, or within a liposomal delivery system. Suitable methods of preparing effervescent compositions containing microencapsulated active ingredients are described, for example, in U.S. Patents 4,462,982, 4,710,384, 5,178,878, and 5,709,886. Preferably, the microencapsulated bisphosphonate has a mean particle size of 25 microns to 120 microns (e.g., 40 microns to 70 microns). More preferably, the microencapsulated bisphosphonate has a mean particle size of 50 microns.

The effervescent compositions described above can be formulated as a tablet, granulate, or a powder. Suitable methods for producing the effervescent compositions of the invention include those described in U.S. Patents 4,687,662, 4,942,039, 5,348,745, 5,415,870, 5,480,652, 5,853,759, and 6,284,272. Preferably, the acid component and the alkaline effervescing component are at least partially reacted with each other during granulation with the bisphosphonate.

The effervescent compositions described above optionally further comprise a colorant. The colorant can be any suitable colorant including natural colorants, food, drug and cosmetics (FD&C) colorants, and drug and cosmetic (D&C) colorants. Suitable natural colorants include red beet powder and beta-carotene powder.

The effervescent compositions described above optionally further comprise other ingredients to aid in the formulation of the composition and/or for aesthetic purposes. Such other ingredients include, for example, fragrances, dyes, filler such as calcium sulfate, starch, and binders. Desirable binders assist in tablet compression and can include starches, pregelatinized starches, gelatin, methylcellulose, sodium carboxymethylcellulose, ethylcellulose, polyacrylamides, polyvinyloxoazolidone, polyvinylalcohols, and polyvinylpyrrolidone.

The effervescent compositions described above optionally further comprise a disintegrant to enhance the disintegration of the compressed tablet in water. The disintegrant can be any suitable disintegrant. For example, the disintegrant can be starch, alginic acid, guar gum, kaolin, bentonite, sodium starch glycolate, isoamorphous silicate, and microcrystalline cellulose.

The effervescent compositions described above optionally further comprise a lubricant, which is applied to dies before the granular mixture is compressed into the effervescent tablet. Lubricants can include hydrogenated or partially hydrogenated vegetable oils such as corn oil, canola oil, cottonseed oil, sesame oil, soybean oil, grape seed oil, sunflower oil, safflower oil, olive oil, peanut oil, and combinations thereof. Lubricants can also include calcium stearate, magnesium stearate, zinc stearate, polyoxyethylene monostearate, talc, polyethyleneglycol, sodium benzoate, sodium lauryl sulfate, magnesium lauryl sulfate, light mineral oil, and combination thereof. Lubricants can form a hydrophobic coating on an effervescent tablet and affect the dissolution rate of the tablet. The preferred embodiment of the invention uses paraffin oil or magnesium stearate as a lubricant. Paraffin oil or magnesium stearate dust imparts desirable dissolution characteristics to the tablets and facilitates the high-speed production of the tablets.

Typically the effervescent compositions of the invention have a total weight of 1500 mg or more (e.g., 2000 mg or more). In some embodiments, the effervescent composition has a total weight of 2500 mg or more. Preferably, effervescent compositions have a total weight of 2000 mg to 6000 mg (e.g., 3500 mg to 5000 mg).

The effervescent compositions of the invention preferably are used for treating and/or preventing conditions or disease states in a mammal involving excessive bone resorption related to bone formation. Such disease states including osteoporosis (e.g., postmenopausal osteoporosis, steroid-induced osteoporosis, male osteoporosis, disease-induced osteoporosis, idiopathic osteoporosis), Paget's disease, abnormally increase bone turnover, periodontal disease, localized bone loss from periprosthetic osteolysis, and bone fractures.

In one embodiment, the effervescent compositions are used in a method of inhibiting bone resorption in a mammal. The treatment comprises combining a bone resorption inhibiting amount of the effervescent composition with water to form at least a partial solution and administering the solution to the mammal (e.g., patient) orally.

In another embodiment, the effervescent compositions are used to treat osteoporosis in a mammal. The treatment comprises combining an osteoporosis-treating effective amount of the effervescent composition with water to form at least a partial solution and administering the solution to the mammal ( e.g., patient) orally.

The administration of the effervescent composition to the mammal preferably causes the pH of the contents of the mammal's stomach (e.g., the gastric juices) to be raised to a pH of 3 or greater. More preferably, the administration of the effervescent composition to the mammal causes the pH of the mammal's stomach to be raised to a pH of 3.5 or greater (e.g., 4 or greater). Typically, the pH of the mammal's stomach is not raised above 6.5 (e.g., not above 6).

The effervescent composition can be administered to a mammal following a continuous schedule. The continuous schedule can be daily, once weekly, twice weekly, thrice weekly, biweekly, twice monthly, or monthly.

The following examples further illustrate the invention.

### EXAMPLE 1

This example illustrates a process for preparing the effervescent tablets of the invention, comprising etidronate and an H₂-antagonist (anti-ulcer agent), using a wet granulation method.

An effervescent tablet of etidronate is formed from the ingredients listed in Table 2.

**Table 2**

| Ingredient | Amount | Function |
|---|---|---|
| etidronate | 70 to 800 mg | active bisphosphonate |
| cimetidine | 100 to 400 mg | anti-ulcer agent |
| citric acid | 800 to 1600 mg | acid component |
| sodium bicarbonate | 800 to 1600 mg | alkaline effervescing cmpd |
| potassium bicarbonate | 0 to 800 mg | alkaline effervescing cmpd |
| sodium carbonate | 20 to 100 mg | alkaline effervescing cmpd |
| polyvinylpyrrolidone | 1 to 5 wt.% by wt | solubilizer |
| polyethylene glycol 6000 | 20 to 100 mg | solubilizer |
| aspartame | 10 to 50 mg | sweetener |
| raspberry flavor | 10 to 100 mg | flavorant |

All process steps for this example are performed under approximately 20% relative humidity. The etidronate, cimetidine, polyvinylpyrrolidone, a part of the citric acid, a part of the sodium bicarbonate, a part of the potassium bicarbonate, a part of the sodium carbonate, and a part of the polyethylene glycol are homogeneously mixed in a high-speed mixer. The powders are wetted with a small amount of water. This wetting initiates a slight effervescent reaction between the acidic and the alkaline components. This effervescent reaction binds the components together with the polyvinylpyrrolidone. The wetted powders are dried with heat in a vacuum at a very low humidity until the powder achieves approximately 0.15 % loss on drying. After sieving for particle size the dried granulate is mixed in a tumbler with the remaining ingredients.

The tablet mixture is compressed on a suitable commercially available rotary tablet press. The dies are lined with nylon inserts and/or lubricated with a thin film of paraffin oil or magnesium stearate, which is applied by spraying. The tablets are filled on-line into tubes or strips.

### EXAMPLE 2

This example illustrates a process for preparing the effervescent tablets of the invention, comprising etidronate and an H₂-antagonist (anti-ulcer agent), using a wet granulation method.

The ingredients listed in Table 3 are used in the production of an effervescent tablet of the invention.

**Table 3**

| Ingredient | Amount Range (in mg) | Actual Amount (in mg) |
|---|---|---|
| etidronate | 70-800 | 200 |
| cimetidine | 100-400 | 250 |
| citric acid | 800-1600 | 1400 |
| sodium bicarbonate | 800-1600 | 800 |
| potassium bicarbonate | 0-800 | 695 |
| sodium carbonate | 20-200 | 160 |
| polyvinylpyrrolidone | 1-5 wt.% | 2 wt.% |
| aspartame | 10-50 | 20 |
| saccharin | 10-30 | 20 |
| polyethylene glycol 6000 | 20-100 | 60 |
| flavorant | 10-100 | 25 |
| colorant | 0-10 | 5 |

The etidronate, polyvinylpyrrolidone, and sodium bicarbonate are premixed. The colorant, cimetidine, and sodium carbonate are premixed. Place the citric acid in a bowl of a suitable blender. Add a sufficient amount of water to the citric acid slowly and mix thoroughly to form a moist blend. Add to the blend, in sequence, while mixing, the sodium bicarbonate mix and the sodium carbonate mix until uniformly distributed. While mixing, the polyethylene glycol is added and the mixture is blended until uniformly distributed.

The tablet mixture is compressed on a suitable commercially available rotary tablet press. The dies are lined with nylon inserts and/or lubricated with a thin film of paraffin oil or magnesium stearate, which is applied by spraying. The tablets are filled on-line into tubes or strips.

### EXAMPLE 3

This example illustrates a process for preparing the effervescent tablets of the invention, comprising etidronate and a proton pump inhibitor (anti-ulcer agent), using a wet granulation method.

The ingredients listed in Table 4 are used in the production of an effervescent tablet of the invention.

**Table 4**

| Ingredient | Actual Amount (in mg) |
|---|---|
| etidronate | 200 |
| omeprazole | 20 |
| citric acid | 420 |
| sodium citrate monobasic | 1820 |
| potassium-sodium tartrate | 5 |
| sodium bicarbonate | 800 |
| potassium bicarbonate | 800 |
| sodium carbonate | 160 |
| polyvinylpyrrolidone | 2 wt.% |
| aspartame | 30 |
| polyethylene glycol | 8000 |
| aspartame | 30 |
| polyethylene glycol 8000 | 45 |
| flavorant | 50 |
| colorant | 5 |

Blend etidronate, omeprazole, citric acid, sodium citrate monobasic, potassium sodium tartrate, polyvinylpyrrolidone, polyethylene glycol, and aspartame in a suitable blender. Quickly add all of water and mix until a workable mass is formed. Granulate through a suitable screen using a granulator. Spread evenly on a paper-lined tray or a fluid bed dryer. Place a dried granulation in a suitable blender and add powder sodium bicarbonate, potassium bicarbonate, and sodium carbonate. Mix well. Add flavorant and colorant and mix until uniformly distributed.

The tablet mixture is compressed on a suitable commercially available rotary tablet press. The process is carried out at room temperature and with a relative humidity not higher than 15-20%. The dies are lined with nylon inserts and/or lubricated with a thin film of paraffin oil or magnesium stearate, which is applied by spraying. The tablets are filled on-line into tubes or strips.

The tablet when dissolved in 120 ml water produces a solution having a buffered pH of 5.5 to 6.0.

### EXAMPLE 4

This example illustrates a process for preparing the effervescent tablets of the invention, comprising etidronate and an H₂-antagonist (anti-ulcer agent), using a dry granulation method.

The ingredients listed in Table 5 are used in the production of an effervescent tablet of the invention.

**Table 5**

| Ingredient | Actual Amount (in mg) |
|---|---|
| etidronate | 200 |
| ranitidine | 200 |
| citric acid | 525 |
| sodium citrate monobasic | 1820 |
| Potassium-sodium tartrate | 5 |
| Sodium bicarbonate | 800 |
| potassium bicarbonate | 695 |
| sodium carbonate | 40 |
| polyvinylpyrrolidone | 2 wt.% |
| Splenda® | 30 |
| polyethylene glycol 8000 | 45 |
| flavorant | 50 |
| colorant | 5 |

Etidronate, ranitidine, polyethylene glycol, and polyvinylpyrrolidone are blended in a suitable mixer until uniform. To the mixture are added citric acid, potassium sodium tartrate, and sodium citrate monobasic, alternating with sodium bicarbonate, potassium bicarbonate, and sodium carbonate. After all the acidic and alkaline components are added, add the remaining ingredients and mix until uniform. Roller compact the powder mix followed by granulation using a suitable screen.

The tablet mixture is compressed on a suitable commercially available rotary tablet press. The process is carried out at room temperature and with a relative humidity not higher than 15-20%. The dies are lined with nylon inserts and/or lubricated with a thin film of paraffin oil or magnesium stearate, which is applied by spraying. The tablets are filled on-line into tubes or strips.

The tablet when dissolved in 120 ml water produces a solution having a buffered pH of 3.0 to 3.4.

### EXAMPLE 5

This example illustrates a process for preparing the effervescent tablets of the invention, comprising etidronate and a proton pump inhibitor (anti-ulcer agent), using a two-part dry granulation method.

The ingredients listed in Table 6 are used in the production of an effervescent tablet of the invention.

**Table 6**

| Ingredient | Actual Amount (in mg) |
|---|---|
| Etidronate | 200 |
| Lansoprazole | 15 |
| citric acid | 475 |
| sodium citrate monobasic | 1820 |
| potassium sodium tartrate | 5 |
| sodium bicarbonate | 800 |
| potassium bicarbonate | 695 |
| sodium bicarbonate | 80 |
| Polyvinylpyrrolidone | 2 wt.% |
| Xylitol | 200 |
| polyethylene glycol 8000 | 45 |
| Flavorant | 50 |
| Colorant | 5 |

In a first mixer, etidronate, lansoprazole, part of the sodium bicarbonate, part of the potassium bicarbonate, polyethylene glycol, and polyvinylpyrrolidone are blended and then the mixture is roller compacted. In a second mixer, citric acid, sodium citrate monobasic, potassium sodium tartrate, the remaining sodium bicarbonate and potassium bicarbonate, sodium carbonate, colorant, flavorant, and xylitol are blended and then roller compacted. The mixtures from the first and second mixer are then granulated through a suitable screen. The remaining ingredients are added and the mixture is blended until uniform.

The tablet mixture is compressed on a suitable commercially available rotary tablet press. The process is carried out at room temperature and with a relative humidity not higher than 15-20%. The dies are lined with nylon inserts and/or lubricated with a thin film of paraffin oil or magnesium stearate, which is applied by spraying. The tablets are filled on-line into tubes or strips.

The tablet when dissolved in 120 ml water produces a solution having a buffered pH of 4.0 to 4.4.

### EXAMPLE 6

This example demonstrates that the bioavailability of etidronate from effervescent tablets is greater than that obtained with non-effervescent etidronate tablets.

A group of 6 beagle dogs is dosed with etridronate following a random three-way design with a two-week washout between each treatment phase. In the first treatment phase (control), the beagle dogs are administered Didronel® (etidronate) tablets (200 mg). In the second and third treatment phases, the beagle dogs are administered effervescent tablets of the invention containing 200 mg etidronate active ingredient in either a buffered or unbuffered formulation, respectively.

For each treatment phase, the bioavailability of etidronate in the beagle dogs is determined. Urine samples are collected after 24 and 48 hours and the concentration of etidronate for each sample is determined by chromatographic analysis. The amount of etidronate found in the urine reflects the percentage of etidronate that was absorbed by the body but did not bind to the surface of the bone. While the amount of etidronate absorbed by the body is dependent on the mode of administration, the percentage of the absorbed etidronate that becomes bound to the bone surface is independent of the mode of drug administration. Thus, an increase in the amount of etidronate in the urine is reflective of an overall increase in absorption.

For the first treatment phase group of beagle dogs (control), the amount of etidronate absorbed into the body is expected to be 3%. For the second and third treatment phase group (invention), the amount of etidronate absorbed into the body is expected to be 6% to 10%. Thus, the bioavailibility of etidronate in treatment phase groups 2 and 3 is expected to at least 50% greater than the bioavailibility from conventional tablet formulations administered to treatment phase group 1.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

## Claims

1. An effervescent composition comprising:
(a) 2 % or more bisphosphonate,
(b) citric acid and
(c) an alkaline effervescing component which is a carbonate salt and/or a bicarbonate salt,
wherein the composition has a total weight of 3500 mg to 6000 mg and when dissolved in water produces a solution having a buffered pH of 4.5 to 6.0 and has buffering capacity sufficient to mediate the pH of a patient's stomach for at least 30 minutes.

2. The composition of claim 1, further comprising a sweetener or flavorant.

3. The composition of claim 1, wherein the alkaline effervescing component is selected from the group consisting of sodium bicarbonate, sodium carbonate anhydrous, potassium carbonate and potassium bicarbonate, sodium glycine carbonate, calcium carbonate, calcium bicarbonate, lysine carbonate, arginine carbonate, and combinations thereof.

4. The composition of claim 1, wherein the acid component and the alkaline effervescing component are at least partially reacted with each other during granulation with the bisphosphonate.

5. The composition of claim 1, further comprising a solubilizing agent.

6. The composition of claim 5, wherein the solubilising agent is selected from the group consisting of polyvinylpyrrolidones, polyethylene glycols, dextrans, and combinations thereof.

7. The effervescent composition of any of the claims 1-6, further comprising an anti-ulcer agent.

8. The effervescent composition of claim 7, wherein the anti-ulcer agent is an H₂-antagonist or a proton pump inhibitor.

9. The composition of claim 8, wherein the H₂-antagonist is present in an amount of from 3.3% to 57.5% by weight of the composition.

10. The composition of claim 8, wherein the H₂-antagonist is selected from the group consisting of ranitidine, cimetidine, famotidine, nizatidine, and combinations thereof.

11. The composition of claim 8, wherein the proton pump inhibitor is present in an amount of from 0.5% to 60% by weight of the composition.

12. The composition of claim 11, wherein the proton pump inhibitor is selected from the group consisting of omeprazole, pantoprazole, lansoprazole, rabeprazole, and combinations thereof.

13. The composition of claim 8, wherein the anti-ucler agent comprises at least one H₂-antagonist and at least one proton pump inhibitor.

## Patentansprüche

1. Sprudelnde Zusammensetzung, umfassend:
(a) 2 % oder mehr Bisphosphonat
(b)Zitronensäure und
(c) eine akalische, sprudelnde Komponente, die ein Carbonatsalz und/oder ein Bicarbonatsalz ist,
wobei die Zusammensetzung ein Gesamtgewicht von 3500 mg bis 6000 mg hat und, wenn sie in Wasser aufgelöst wird, eine Lösung produziert, die einen gepufferten pH von 4,5 bis 6,0 hat und die eine ausreichende Pufferkapazität besitzt, um den pH des Magens eines Patienten für mindestens 30 Minuten auszugleichen.

2. Zusammensetzung nach Anspruch 1, weiterhin umfassend einen Süssstoff oder einen Geschmacksstoff.

3. Zusammensetzung nach Anspruch 1, wobei die akalische, sprudelnde Komponente Natriumbicarbonat, wasserfreies Natriumcarbonat, Kaliumcarbonat, Kaliumbicarbonat, Natriumglycincarbonat, Calciumcarbonat, Calciumbicarbonat, Lysincarbonat und/oder Arginincarbonat ist.

4. Zusammensetzung nach Anspruch 1, wobei die saure Komponente und die akalische, sprudelnde Komponente während der Granulierung mit dem Bisphosphonat zumindest teilweise miteinander umgesetzt werden.

5. Zusammensetzung nach Anspruch 1, weiterhin umfassend ein lösendes Mittel.

6. Zusammensetzung nach Anspruch 5, wobei das lösende Mittel Polyvinylpyrrolidon, Polyethylenglykol und/oder Dextran ist.

7. Sprudelnde Zusammensetzung nach irgendeinem der Ansprüche 1 bis 6, weiterhin umfassend ein Anti-Ulcusmittel.

8. Sprudelnde Zusammensetzung nach Anspruch 7, wobei das Anti-Ulcusmittel ein H₂-Antagonist oder ein Protonenpumpeninhibitor ist.

9. Zusammensetzung nach Anspruch 8, wobei der H₂-Antagonist in einer Menge im Bereich von 3,3 Gew.-% bis 57,5 Gew.-% der Zusammensetzung vorliegt.

10. Zusammensetzung nach Anspruch 8, wobei der H₂-Antagonist Ranitidin, Cimetidin, Famotidin und/oder Nizatidin ist.

11. Zusammensetzung nach Anspruch 8, wobei der Protonenpumpeninhibitor in einer Menge im Bereich von 0,5 Gew.-% bis 60 Gew.-% der Zusammensetzung vorliegt.

12. Zusammensetzung nach Anspruch 11, wobei der Protonenpumpeninhibitor Omeprazol, Pantoprazol, Lansoprazol und/oder Rabeprazol ist.

13. Zusammensetzung nach Anspruch 8, wobei das Anti-Ulcusmittel mindestens einen H₂-Antagonisten und mindestens einen Protonenpumpeninhibitor umfasst.

## Revendications

1. La composition effervescente comprenant :
(a) 2 % ou plus de 2 % bisphosphonate
(b) de l'acide citrique et
(c) un composant alcalin effervescent qui est un sel de carbonate et/ou un sel de bicarbonate,
dans laquelle la composition possède un poids total de 3500 mg à 6000 mg et, quand elle est dissoute dans de l'eau, elle produit une solution ayant un pH tamponné de 4,5 à 6,0 et possède une capacité tampon suffisante pour influencer le pH de l'estomac d'un patient pendant au moins 30 minutes.

2. La composition selon la revendication 1, comprenant en outre un édulcorant ou un aromatisant.

3. La composition selon la revendication 1, dans laquelle le composant alcalin effervescent est choisi dans le groupe constitué par le bicarbonate de sodium, le carbonate de sodium anhydre, le carbonate de potassium et le bicarbonate de potassium, le carbonate de glycine sodique, le carbonate de calcium, le bicarbonate de calcium, le carbonate de lysine, le carbonate d'arginine et leurs combinaisons.

4. La composition selon la revendication 1, dans laquelle le composant acide et le composant alcalin effervescent réagissent au moins partiellement l'un avec l'autre pendant la granulation avec le bisphosphonate.

5. La composition selon la revendication 1, comprenant en outre un agent solubilisant.

6. La composition selon la revendication 5, dans laquelle l'agent solubilisant est choisi dans le groupe constitué par les polyvinylpyrrolidones, les polyéthylèneglycols, les dextranes et leurs combinaisons.

7. La composition effervescente selon l'une quelconque des revendications 1 à 6, comprenant en outre un agent anti-ulcère.

8. La composition effervescente selon la revendication 7, dans laquelle l'agent anti-ulcère est un antagoniste H₂ ou un inhibiteur de pompe à proton.

9. La composition selon la revendication 8, dans laquelle l'antagoniste H₂ est présent en une quantité de 3,3 % à 57,5 % en poids de la composition.

10. La composition selon la revendication 8, dans laquelle l'antagoniste H₂ est choisi dans le groupe constitué par la ranitidine, la cimétidine, la famotidine, la nizatidine et leurs combinaisons.

11. La composition selon la revendication 8, dans laquelle l'inhibiteur de pompe à proton est
présent en une quantité de 0,5 % à 60 % en poids de la composition.

12. La composition selon la revendication 11, dans laquelle l'inhibiteur de pompe à proton est choisi dans le groupe constitué par l'oméprazole, le pantoprazole, le lansoprazole, le rabéprazole et leurs combinaisons.

13. La composition selon la revendication 8, dans laquelle l'agent anti-ulcère comprend au moins un antagoniste H₂ et au moins un inhibiteur de pompe à proton.
